(19) Europäisches Patentamt
European Patent Office
Office européen des brevets

(11) Veröffentlichungsnummer: **0 359 117 A2**

# (12) EUROPÄISCHE PATENTANMELDUNG

(21) Anmeldenummer: **89116512.8**

(22) Anmeldetag: **07.09.89**

(51) Int. Cl.5: **C07C 43/188 , C07C 41/08**

(30) Priorität: **16.09.88 DE 3831485**

(43) Veröffentlichungstag der Anmeldung:
**21.03.90 Patentblatt 90/12**

(84) Benannte Vertragsstaaten:
**CH DE FR GB LI NL**

(71) Anmelder: **BASF Aktiengesellschaft**
**Carl-Bosch-Strasse 38**
**D-6700 Ludwigshafen(DE)**

(72) Erfinder: **Lauterbach, Gerald, Dr.**
**Arminstrasse 38 a**
**D-6140 Bensheim(DE)**
Erfinder: **Eckhardt, Heinz, Dr.**
**Ruedigerstrasse 7**
**D-6700 Ludwigshafen(DE)**
Erfinder: **Schindler, Gerhard**
**Collinistrasse 5**
**D-6800 Mannheim 1(DE)**
Erfinder: **Pape, Frank-Friedrich, Dr.**
**Berner Weg 34**
**D-6700 Ludwigshafen(DE)**
Erfinder: **Becker, Rainer, Dr.**
**Im Haseneck 22**
**D-6702 Bad Duerkheim(DE)**

## (54) Cycloalkylvinylether, deren Herstellung und Verwendung.

(57) Cycloalkylvinylether der allgemeinen Formel I

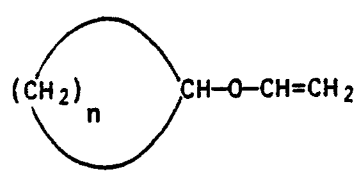

(I),

in der n für eine ganze Zahl von 7 bis 15 steht, insbesondere Cyclododecylvinylether, deren Herstellung und Verwendung.

EP 0 359 117 A2

## Cycloalkylvinylether, deren Herstellung und Verwendung

Die Erfindung betrifft Cycloalkylvinylether der allgemeinen Formel I

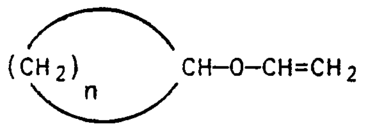

(I),

in der n für eine ganze Zahl von 7 bis 15 steht, insbesondere Cyclododecylvinylether, sowie deren Herstellung und Verwendung.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an Geruchsverbesserern für Produkte des täglichen Bedarfs, wie Reinigungsmitteln, Kosmetika, Leimen etc. hat die Riechstoffindustrie ständig Bedarf an neuen Riechstoffen, die allein oder in Form von Kompositionen wertvolle Parfüms bzw. Duftstoffe mit interessanten Duftnoten darstellen. Da aufgrund wenig bekannter Zusammenhänge zwischen Struktur und Riechstoffeigenschaften eine gezielte Synthese von Riechstoffen mit gewünschten olfaktorischen Eigenschaften nicht möglich ist, besteht die Aufgabe, solche Verbindungen aufzufinden, die wertvolle Riechstoffqualitäten besitzen.

Es war daher die Aufgabe der Erfindung, neue interessante Riechstoffe zu entwickeln, die auf möglichst einfache Weise aus gut zugänglichen und daher billigen Ausgangsstoffen hergestellt werden können.

Aus der DE-AS 1 196 810 sind aliphatische Cyclododecylether als Riechstoffe mit wertvollen Holznoten bekannt. Als besonders interessant erwies sich der Cyclododecylethylether wegen seines reinen Zedernholzgeruches. Einer breiten Verwendung der beschriebenen Riechstoffe steht ein technisch schwierig durchzuführendes Verfahren, das zu einer großen Abwasserbelastung führt, entgegen. Gemäß dem in loc. cit. beschriebenen Herstellungsverfahren wird aus Cyclododecanol mit Hilfe des schwierig zu handhabenden Natriumamids das Alkoholat hergestellt, wobei in stöchiometrischen Mengen Ammoniak anfällt, der zum Ammoniumsalz umgesetzt werden muß und in dieser Form ins Abwasser gelangt. Das erhaltene Alkoholat wird anschließend mit hochtoxischen Alkylierungsmitteln, wie Dialkylsulfaten, in einer stark exothermen Reaktion zum entsprechenden Alkylether umgesetzt. Hierbei fällt erneut in überstöchiometrischer Menge Salz als Abfallprodukt an.

Es war daher auch eine Aufgabe der Erfindung, den als Riechstoff sehr begehrten Cyclododecylethylether auf technisch einfache Weise und mit möglichst geringer Umweltbelastung herzustellen.

Es wurde nun gefunden, daß man den Cyclododecylethylether auch in technischem Maßstab auf einfache Weise und mit sehr guten Ausbeuten erhalten kann, wenn man Cyclododecanol in Gegenwart basischer Katalysatoren mit Acetylen umsetzt und den dabei erhaltenen Cyclododecylvinylether anschließend katalytisch zu dem Cyclododecylethylether hydriert.

Durch dieses Verfahren gelingt die Darstellung des begehrten Cyclododecylethylethers sowie anderer Cycloalkylethylether mit Hilfe technisch gut durchführbarer katalytischer Reaktionen, bei deren Durchführung toxische Stoffe vermieden werden und eine Umweltbelastung durch Salzanfall ausgeschlossen wird. Da außerdem auf Verwendung organischer Lösungsmittel sowie ein Waschen der Reaktionsausträge mit Wasser verzichtet werden kann, ist das vorliegende Verfahren ein besonders umweltschonender und kostengünstiger Weg zur Herstellung des gewünschten Cyclododecylethylethers.

Überraschenderweise wurde nun gefunden, daß der bei diesem Prozeß als Zwischenprodukt auftretende Cyclododecylvinylether auch wertvolle Riechstoffeigenschaften aufweist. Cyclododecylvinylether weist eine frische, etwas animalische und eine - im Vergleich zu den bekannten Cyclododecylethern - weitaus weniger holzige Geruchsnote auf. Im Gegensatz zu den bekannten Cyclododecylethern kann der neue Cyclododecylvinylether daher außer in Edelholzkompositionen auch in vielen anderen Kompositionen eingesetzt werden. Einen überraschend positiven Effekt bewirkt Cyclododecylvinylether u.a. in den häufig eingesetzten blumigen Kompositionen. Insbesondere bewirkt Cyclododecylvinylether eine deutliche Anhebung der Komplexität und Intensität des Duftakkords. Außerdem ist eine abrundende Wirkung insbesondere auf die holzigen und grün-krautigen Teilaspekte dieser Dufttypen festzustellen. Die Verbindung hat zudem sehr gute fixierende Eigenschaften und ist außerdem im alkalischen pH-Bekreich, dem weitaus häufigsten Einsatzbereich für Duftstoffe, sehr stabil.

Auch andere Cycloalkylvinylether weisen wertvolle Riechstoffeigenschaften auf. Gegenstand der Erfindung sind daher die Cycloalkylvinylether der allgemeinen Formel I

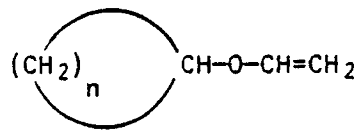

(I),

in der n für eine ganze Zahl von 7 bis 15 steht, deren Verwendung zur Verleihung, Verbesserung oder Modifizierung der Duftstoffeigenschaften von Parfüms oder parfümierten PÖrodukten sowie ein Verfahren zu deren Herstellung, das dadurch gekennzeichnet ist, daß man Cycloalkanole der allgemeinen Formel II

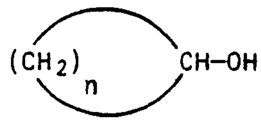

(II),

in der n für eine ganze Zahl von 7 bis 15 steht, in Gegenwart basischer Katalysatoren mit Acetylen umsetzt.

Gegenstand der Erfindung ist weiterhin die Verwendung von Cyclododecylvinylether zur Herstellung des begehrten Cyclododecylethylethers sowie allgemein ein Verfahren zur Herstellung von Cyclododecylethylethern der allgemeinen Formel III

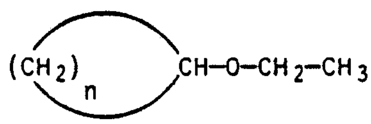

(III),

das dadurch gekennzeichnet ist, daß man die gemäß dem oben beschriebenen Verfahren erhaltenen Cycloalkylvinylether der Formel (I) katalytisch hydriert.

Als basische Katalysatoren für die erfindungsgemäße Umsetzung mit Acetylen sind insbesondere geeignet Alkalialkoholate, wie Natriummethylat Kaliummethylat und Kalium-tert.-butylat oder Alkalihydroxyde, wie Kaliumhydroxid und Natriumhydroxid.

Im allgmeinen arbeitet man mit Acetylendrucken von 1 bis 20, vorzugsweise 5 bis 12 bar und Reaktionstemperaturen von 50 bis 250, vorzugsweise 130 bis 180 °C.

Als Hydrierungskatalysatoren sind Metallkatalysatoren, wie Nickel- und Kobaltkatalysatoren, insbesondere Edelmetallkatalysatoren, wie Platin-, Palladium-, Ruthenium- und Rhodium-Katalysatoren geeignet. Insbesondere seien Raney-Nickel, Palladium- und Platin-trägerkatalysatoren genannt.

Im allgemeinen arbeitet man bei der Hydrierung mit $H_2$-Drucken von 1 bis 200, vorzugsweise 10 bis 60 bar und Reaktionstemperaturen von 0 bis 150, vorzugsweise 20 bis 60 °C.

Die Cycloalkylvinylether der Formel I, insbesondere Cyclododecylvinylether, stellen wertvolle Riechstoffe dar, die auf technisch einfache und vorteilhafte Weise hergestellt werden können. Cyclododecylvinylether eröffnet außerdem einen sehr vorteilhaften Weg zu dem als Riechstoff mit edlem Zedernholzduft begehrten Cyclododecylethylether.

Beispiel 1

Herstellung von Cyclododecylvinylether

Ein Gemisch aus 1840 g Cyclododecanol und 32 g pulverisiertem KOH wurde in einem Rührautoklaven vorgelegt. Anschließend wurde der Autoklav mit Stickstoff gespült und bei 2 bar $N_2$ auf 160 °C aufgeheizt. Man preßte dann 22 bar Acetylen auf und kompensierte den Verbrauch durch ständiges Nachpressen von Acetylen. Nach 12 h war keine Gasaufnahme mehr zu beobachten. Der Autoklav wurde entspannt und abgekühlt. Nach Destillation unter vermindertem Druck erhielt man 1827 g Cyclododecylvinylether bei Kp. 135-138 °C/1 mbar (entsprechend einer Ausbeute von 87 % d. Theorie).

Beispiel 2

Herstellung von Cyclododecylethylether

413 g Cyclododecylvinylether und 2 g Pd/C (5 %ig) wurden im Hydrierautoklaven gemischt. Anschließend wurde bei 50 °C und 50 bar $H_2$ so lange hydriert bis keine $H_2$-Aufnahme mehr erfolgte. Nach dem Abkühlen wurde der Katalysator abfiltriert und das Filtrat destilliert. Man erhielt 405 g Cyclododecylethylether (Kp. 98-102 °C/0,15 mbar) (97 % der Theorie).

Beispiel 3 (Anwendungsbeispiel)

In der Komposition I, Typ "Rose", der unten angegebenen Zusammensetzung wurden 100 Gewichtsteile Dipropylenglykol durch den erfindungsgemäßen Cyclododecylvinylether ersetzt (Ia)

| | I | Ia |
|---|---|---|
| Geraniumoel Bourbon | 5 Gew.-Teile | 5 Gew.-Teile |
| Phenylethylisoamylether | 10 Gew.-Teile | 10 Gew.-Teile |
| Citral 10 % in DPG | 20 Gew.-Teile | 20 Gew.-Teile |
| Phenylacetaldehyd 50, 10 % in DPG | 20 Gew.-Teile | 20 Gew.-Teile |
| Dihydrorosenoxid 10 % in DPG | 30 Gew.-Teile | 30 Gew.-Teile |
| Phenylethylacetat | 40 Gew.-Teile | 40 Gew.-Teile |
| Geranylacetat | 50 Gew.-Teile | 50 Gew.-Teile |
| Phenylethylalkohol | 80 Gew.-Teile | 80 Gew.-Teile |
| Dipropylenglykol (DPG) | 195 Gew.-Teile | 95 Gew.-Teile |
| Cyclododecylvinylether/BASF | --- Gew.-Teile | 100 Gew.-Teile |
| Geraniol | 250 Gew.-Teile | 250 Gew.-Teile |
| Citronellol | 300 Gew.-Teile | 300 Gew.-Teile |
| | 1000 Gew.-Teile | 1000 Gew.-Teile |

Die beschriebene Riechstoffkomposition erhält durch Zufügen des erfindungsgemäßen Cyclododecylvinylethers einen wesentlich intensiveren Duft. Die holzigen und grün-krautigen Teilaspekte des Duftes wurden abgerundet.

Beispiel 4 (Anwendungsbeispiel)

In der Komposition II, Typ "Violet", der unten angegebenen Zusammensetzung wurden anstelle von 100 Gew.-teilen Dipropylenglykol 100 Gew.-teile des erfindungsgemäßen Cyclododecylvinylethers (IIa) zugefügt.

| | II | IIa |
|---|---|---|
| cis-3-Hexen-1-ol | 10 Gew.-teile(%) | 10 Gew.-teile(%) |
| Heliotropin | 10 Gew.-teile(%) | 10 Gew.-teile(%) |
| Dihydromyrcenol | 20 Gew.-teile(%) | 20 Gew.-teile(%) |
| Benzylacetat | 30 Gew.-teile(%) | 30 Gew.-teile(%) |
| Hydroxycitronellol | 50 Gew.-teile(%) | 50 Gew.-teile(%) |
| Canthoxal | 50 Gew.-teile(%) | 50 Gew.-teile(%) |
| Linalylacetat | 60 Gew.-teile(%) | 60 Gew.-teile(%) |
| Dimethylphenylethylcarbinol | 70 Gew.-teile(%) | 70 Gew.-teile(%) |
| Dipropylenglykol | 100 Gew.-teile(%) | --- Gew.-teile(%) |
| Cyclododecylvinylether/BASF | --- Gew.-teile(%) | 100 Gew.-teile(%) |
| alpha-Hexylzimtaldehyd | 100 Gew.-teile(%) | 100 Gew.-teile(%) |
| Phenylethylalkohol | 100 Gew.-teile(%) | 100 Gew.-teile(%) |
| alpha-Ionon | 400 Gew.-teile(%) | 400 Gew.-teile(%) |
| | 1000 Gew.-teile | 1000 Gew.-teile |

Die beschriebene Riechstoffkomposition II erhielt durch Zufügen des erfindungsgemäßen Cyclododecylvinylethers einen wesentlich abgerundeten und intensiveren Duft.

## Ansprüche

1. Cycloalkylvinylether der allgemeinen Formel I

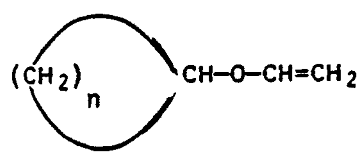

(I),

in der n für eine ganze Zahl von 7 bis 15 steht.

2. Cyclododecylvinylether.

3. Verfahren zur Herstellung von Cycloalkylvinylethern der allgemeinen Formel I gemäß Anspruch 1, dadurch gekennzeichnet, daß man Cycloalkanole der allgemeinen Formel II

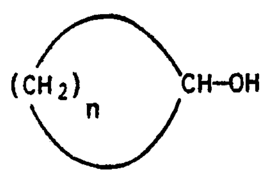

(II),

in der n für eine ganze Zahl von 7 bis 15 steht, in Gegenwart basischer Katalysatoren mit Acetylen umsetzt.

4. Verwendung von Cycloalkylvinylethern der allgemeinen Formel I gemäß Anspruch 1 zur Verleihung, Verbesserung oder Modifizierung der Dufteigenschaften von Parfüms oder parfümierten Produkten.

5. Verwendung von Cyclododecylvinylether gemäß Anspruch 2 zur Verleihung, Verbesserung oder Modifizierung der Dufteigenschaften von Parfüms oder parfümierten Produkten.

6. Verfahren gemäß Anspruch 3, dadurch gekennzeichnet, daß man die erhaltenen Cycloalkylvinylether der Formel I anschließend katalytisch zu den Cycloalkylethylethern der allgemeinen Formel III

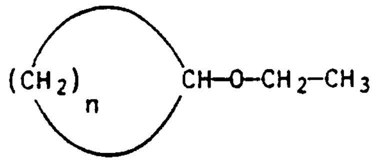

(III),

in der n für eine ganze Zahl von 7 bis 15 steht, hydriert.

7. Verwendung von Cyclododecylvinylether für die Herstellung von Cyclododecylethylether.